# FASCICULE DE BREVET EUROPEEN

(11) **EP 0 550 330 B1**
(45) Date de publication et mention de la délivrance du brevet: **03.03.1999**
(21) Numéro de dépôt: 92403536.3
(22) Date de dépôt: 23.12.1992
(51) Int. Cl.: A61M 25/04

(54) **Dispositif de canulation ventriculaire**
Herzkammer-Kanüliervorrichtung
Ventricular cannulation device

(30) Priorité: 23.12.1991 FR 9115981
(43) Date de publication de la demande: 07.07.1993
(73) Titulaire: ELA MEDICAL (Société anonyme), F-92541 Montrouge Cédex (FR); INSTITUT NATIONAL DE LA SANTE ET DE LA RECHERCHE MEDICALE (INSERM), 75654 Paris Cédex 13 (FR)
(72) Inventeur: Paillot, René, F-94310 Orly (FR); Barra, Jean-Aubert, F-29200 Brest (FR)
(74) Mandataire: Laget, Jean-Loup

(56) Documents cités:
- WO-A-82/01644
- US-A- 2 638 901
- US-A- 3 540 451
- US-A- 4 368 736

## Description

L'invention concerne un dispositif de canulation ventriculaire.

Dans certains cas d'insuffisance cardiaque il est connu de prélever du sang dans le ventricule gauche pour le ré-injecter dans l'aorte, éventuellement après passage dans une pompe.

Ce prélèvement sanguin est effectué en faisant remonter dans l'aorte un tube dont l'extrémité est fixée dans le ventricule gauche. Ce tube est introduit en un point déterminé de l'aorte et, après passage dans la pompe, le sang est ré-injecté en un point de l'aorte situé en aval du point d'introduction du tube (voir document WO 82/01644).

Selon une nouvelle méthode, le sang est prélevé dans le ventricule gauche directement à travers la paroi cardiaque, et il circule dans une prothèse vasculaire, pour être ensuite ré-injecté dans l'aorte.

L'établissement d'une communication à travers la paroi cardiaque pose un certain nombre de problèmes liés au fait que la paroi, qui a une épaisseur de l'ordre de 1,5cm, est toujours en mouvement.

Il est en particulier indispensable que cette communication soit établie dans des conditions rigoureuses d'étanchéité.

Le but de l'invention est de proposer un dispositif assurant une communication à travers la paroi cardiaque, entre la cavité cardiaque et une prothèse vasculaire, avec une sécurité suffisante pour satisfaire à la condition d'étanchéité.

L'invention à pour objet un dispositif de canulation ventriculaire, pour assurer une communication à travers la paroi cardiaque, entre la cavité cardiaque et une prothèse vasculaire, caractérisé en ce qu'il comporte :
- une partie interne, adaptée à être placée dans la cavité cardiaque,
- une partie externe, adaptée à être placée à l'extérieur de la paroi cardiaque,
- et un tube correspondant à l'orifice de la paroi cardiaque et servant de support à ladite prothèse vasculaire,
- lesdites deux parties étant munies chacune de moyens d'accrochage à la paroi cardiaque, pénétrant dans la paroi cardiaque
- ladite partie interne portant des moyens de guidage et de fixation de ladite partie externe, distincts du tube,
- ledit tube étant porté par la partie interne (1) ou par la partie externe (2).

Selon d'autres caractéristiques de l'invention :
- la partie interne est en forme de couronne et ses moyens d'accrochage sont en forme de griffes,
- les griffes s'étendent radialement autour de la couronne et portent chacune un tampon d'appui sur la paroi cardiaque,
- les griffes portent une résille en tissu biocompatible,
- les griffes sont rabattables dans l'encombrement de la couronne pour l'introduction de la partie interne dans la cavité cardiaque,
- le tube est solidaire de la couronne,
- la partie externe est en forme de disque et ses moyens d'accrochage sont des aiguilles perpendiculaires au disque,
- le disque porte le tube,
- les moyens de guidage et de fixation de la partie externe sont constitués par des tiges susceptibles d'être bloquées, de préférence par sertissage, et coupées après mise en place du dispositif sur la paroi cardiaque,
- l'orifice central de la couronne est obturé, pendant la mise en place du dispositif, par un bouchon solide ou gonflable, amovible.

D'autres caractéristiques ressortent de la description qui suit faite avec référence au dessin annexé sur lequel on peut voir.
Fig. 1 : une vue schématique en coupe axiale d'un premier mode de réalisation du dispositif de canulation ventriculaire selon l'invention ;
Fig. 2 : une vue schématique en coupe axiale d'un deuxième mode de réalisation du dispositif de canulation ventriculaire selon l'invention ;
Fig. 3 : un schéma de principe, en coupe axiale, de la disposition relative de la paroi cardiaque et du dispositif de canulation selon l'invention ;
Fig. 4 : un schéma simplifié d'un mode de réalisation de la partie interne du dispositif de canulation avant son introduction dans le ventricule.

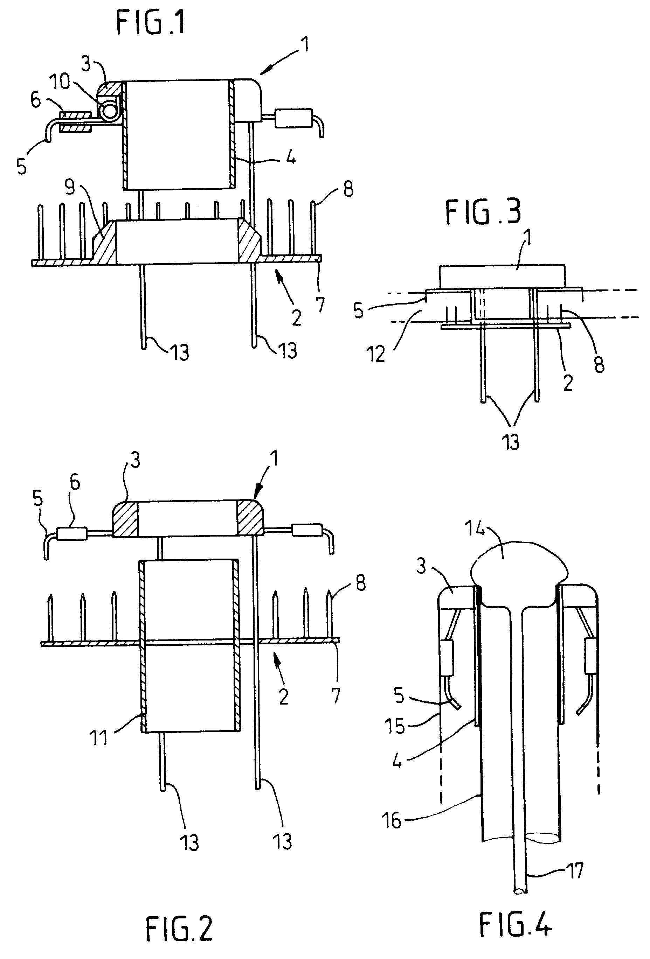

Sur la Fig. 1, le dispositif de canulation ventriculaire selon l'invention se compose essentiellement de deux parties : une partie interne 1 destinée à être placée dans la cavité ventriculaire, et une partie externe 2 destinée à rester hors de la paroi ventriculaire. La partie interne 1 se compose d'une couronne 3 et d'une portion de tube 4 disposée à l'intérieur de la couronne et faisant saillie vers l'extérieur de la cavité ventriculaire. Le tube 4 porte la prothèse vasculaire (16, Fig. 4) qui est fixée, par exemple par collage, soit à l'intérieur, soit à l'extérieur du tube 4. Dans la couronne 3 sont disposées des griffes 5 tournées vers la paroi cardiaque et qui constituent des moyens d'accrochage de la partie interne 1 à la paroi cardiaque.

Ces griffes sont constituées par un fil métallique élastique formant une spire 10, dans l'épaisseur de la couronne 3, et portant dans sa partie extérieure sensiblement rectiligne un tampon 6 destiné à s'appuyer sur la paroi cardiaque avec une surface relativement large.

La partie externe 2 se présente sous forme d'un disque 7 portant des aiguilles 8 tournées vers la paroi cardiaque et qui constituent des moyens d'accrochage de la partie externe 2 à la paroi cardiaque. Le disque 7 présente un orifice central suffisant pour laisser passer le tube 4. Autour de cet orifice central est prévu un manchon 9 sensiblement cylindrique, destiné à recouvrir en partie le tube 4. Le manchon 9 permet le passage du tube 4 et de la prothèse vasculaire 16 avec glissement souple.

Les deux parties 1 et 2 sont mises en place respectivement l'une à l'intérieur de la cavité ventriculaire, l'autre à l'extérieur, et elles sont fixées l'une à l'autre en maintenant entre elles la paroi cardiaque grâce aux griffes 5 et aux aiguilles 8. La paroi cardiaque est confinée autour du tube 4 qui assure le passage du sang ventriculaire.

Le fil métallique constituant les griffes 5 est fixé à la partie supérieure (sur la fig. 1) de la couronne 3. Il est enroulé pour constituer une spire 10 et pour s'écarter radialement par rapport à l'axe de la couronne 3. Au repos, l'ensemble des griffes constitue une sorte de parapluie ouvert, et chaque griffe tend à reprendre sa position de repos lorsqu'elle a été sollicitée mécaniquement hors de cette position.

Avantageusement, l'ensemble des griffes 5 porte une résille en tissu biocompatible, qui se déploie comme le voile d'un parapluie, pour assurer une liaison plus intime avec la paroi cardiaque. De plus, pour éviter un effet thrombogène, la partie interne 1 peut être recouverte d'un revêtement biocompatible.

Sur la Fig. 2, la partie interne 1 présente la même couronne 3 et les mêmes griffes que sur la Fig. 1, mais ne porte plus le tube 4. La partie externe 2 comporte le même disque 7 et les mêmes aiguilles 8 que sur la Fig. 1, mais ne porte plus le manchon 9. Le tube 11 est solidaire du disque 7 et sert de support au tube extérieur.

Sur la Fig. 3, on voit les deux parties 1 et 2 du dispositif placées de part et d'autre de la paroi cardiaque 12 qui a été préalablement incisée.

La paroi cardiaque présente alors un orifice circulaire, dont le diamètre correspond sensiblement à celui du tube (4 ou 11). La partie interne 1 est placée dans la cavité cardiaque, et la partie externe 2 en dehors de cette cavité. Les aiguilles 8 et les griffes 5 pénètrent dans la paroi cardiaque pour la fixer de façon efficace.

Pour serrer l'une vers l'autre les deux parties 1 et 2 du dispositif, des tiges 13 sont prévues, par exemple au nombre de trois. Les tiges 13 solidaires de la partie interne 1, traversent la partie externe 2 en passant dans des orifices prévus à cet effet, et dont le diamètre est juste suffisant pour laisser passer les tiges. De cette façon, lorsque la partie interne 1 est pratiquement mise en place, la partie externe 2 est à son tour mise en place par coulissement sur les tiges 13. Lorsque les deux parties 1 et 2 enserrent la paroi cardiaque et la maintiennent de façon satisfaisante, les tiges 13 sont bloquées au droit du disque 7 par sertissage ou écrasement par exemple, puis coupées.

Pour faire pénétrer la partie interne 1 dans la cavité cardiaque, on dispose tout d'abord dans son orifice axial un bouchon 14, solide ou gonflable, qui assure l'obturation de l'orifice central (Fig. 4). Par ailleurs, les griffes 5 sont rabattues vers le tube 4 ou 11, et un cylindre 15 est mis en place, dont le diamètre extérieur correspond au diamètre de la couronne 3.

Ce cylindre a pour rôle de maintenir les griffes 5 rabattues, dans l'encombrement de la couronne 3, de façon à leur faire franchir la paroi cardiaque 12. Lorsque le bord supérieur du cylindre 15 est dans la cavité cardiaque, on fait progresser la couronne 3 vers l'intérieur de la cavité, par exemple par action sur les tiges 13, de façon à libérer les griffes 5 qui prennent leur position en parapluie ouvert. Le cylindre 15 est ensuite enlevé. On met alors en place la partie externe 2 que l'on fixe, par exemple par sertissage, et on coupe les tiges 13. Avantageusement, la partie externe 2 est mise en position d'attente sur les tiges 13 avant l'intervention, ainsi qu'un outil prévu pour assurer la progression de la partie externe 2 vers la paroi cardiaque, le long des tiges 13.

Sur la Fig. 4, le bouchon 14 représenté est de type gonflable. Son tube de gonflage 17 est disposé à l'intérieur de la prothèse vasculaire 16, et les moyens de gonflage et de dégonflage sont prévus à distance.

Pour assurer le prélèvement sanguin dans la cavité cardiaque, on retire le bouchon 14, le cas échéant après l'avoir dégonflé.

Chacune des parties 1 et 2 du dispositif de canulation ventriculaire peut avantageusement porter sur sa face d'appui contre la paroi cardiaque, un anneau ou une bague en matériau biocompatible présentant une certaine souplesse. Cet anneau, ou cette bague, joue le rôle d'anneau d'étanchéité et joue également un rôle d'amortisseur lors de la mise en place du dispositif, pour limiter l'écrasement de la paroi cardiaque.

## Revendications

1. Dispositif de canulation ventriculaire, pour assurer une communication à travers la paroi cardiaque, entre la cavité cardiaque et une prothèse vasculaire, caractérisé en ce qu'il comporte :
- une partie interne (1), adaptée à être placée dans la cavité cardiaque,
- une partie externe (2), adaptée à être placée à l'extérieur de la paroi cardiaque,
- et un tube (4, 11) correspondant à l'orifice de la paroi cardiaque et servant de support à ladite prothèse vasculaire,
- lesdites deux parties (1, 2) étant munies chacune de moyens d'accrochage (5, 8) à la paroi cardiaque, pénétrant dans la paroi cardiaque
- ladite partie interne (1) portant des moyens (13) de guidage et de fixation de ladite partie externe (2), distincts du tube,
- ledit tube étant porté par la partie interne (1) ou par la partie externe (2).

2. Dispositif selon la revendication 1, caractérisé en ce que la partie interne (1) est en forme de couronne (3) et ses moyens d'accrochage sont en forme de griffes (5).

3. Dispositif selon la revendication 2, caractérisé en ce que les griffes s'étendent radialement autour de la couronne (3) et portent chacune un tampon (6) d'appui sur la paroi cardiaque.

4. Dispositif selon la revendication 2, caractérisé en ce que les griffes (5) portent une résille en tissu biocompatible.

5. Dispositif selon la revendication 2, caractérisé en ce que les griffes (5) sont rabattables dans l'encombrement de la couronne (3) pour l'introduction de la partie interne (1) dans la cavité cardiaque.

6. Dispositif selon la revendication 2, caractérisé en ce que le tube (4) est solidaire de la couronne (3)

7. Dispositif selon la revendication 1, caractérisé en ce que la partie externe (2) est en forme de disque (7) et ses moyens d'accrochage sont des aiguilles (8) perpendiculaires au disque (7).

8. Dispositif selon la revendication 7, caractérisé en ce que le disque (7) porte le tube (11).

9. Dispositif selon la revendication 1, caractérisé en ce que les moyens de guidage et de fixation de la partie externe (2) sont constitués par des tiges (13) susceptibles d'être bloquées, de préférence par sertissage, et coupées après mise en place du dispositif sur la paroi cardiaque.

10. Dispositif selon la revendication 2, caractérisé en ce que l'orifice central de la couronne (3) est obturé, pendant la mise en place du dispositif, par un bouchon (14) solide ou gonflable, amovible.

## Claims

1. A ventricular cannulation device for providing communication through the cardiac wall, between the cardiac chamber and a vascular prosthesis, characterised in that it comprises :
- an inner part (1) adapted to be placed in the cardiac chamber,
- an outer part (2) adapted to be placed outside the cardiac wall,
- and a tube (4, 11) corresponding to the opening of the cardiac wall and serving as a support for said vascular prosthesis,
- each of said two parties (1, 2) being provided with means for catching (5, 8) onto the cardiac wall and penetrating into the same,
- said inner part (1) supporting means (13) for guiding and attaching said outer part (2), which means are separate from the tube,
- said tube being supported by the inner part (1) or by the outer part (2).

2. The device according to claim 1, characterised in that the inner part (1) has the shape of a crown (3) and that its catching means is in the form of claws (5).

3. The device according to claim 2, characterised in that the claws extend radially around said crown (3) and that each claw has a pad (6) for resting against the cardiac wall.

4. The device according to claim 2, characterised in that the claws (5) carry a netting in biocompatible material.

5. The device according to claim 2, characterised in that the claws (5) can be pulled down into the hollow space of the crown (3) for inserting said inner part (1) into said cardiac chamber.

6. The device according to claim 2, characterised in that said tube (4) is interdependent with said crown (3).

7. The device according to claim 1, characterised in that the outer part (2) has the shape of a disk (5) and that its catching means are needles (8) perpendicular to the disk (7).

8. The device according to claim 7, characterised in that the disk (7) bears the tube (11).

9. The device according to claim 1, characterised in that the means for guiding and attaching the outer part (2) is constituted by rods (13) that can be blocked, preferably by crimping, and cut after positioning of the device on the cardiac wall.

10. The device according to claim 2, characterised in that the central opening of the crown (3) is occluded, during the positioning of the device, by a removable solid or inflatable plug.

## Patentansprüche

1. Vorrichtung für eine Herzkammerkanüle zum Gewährleisten einer Verbindung zwischen dem Herzhohlraum und einer Gefäßprothese durch die Herzwand,
**dadurch gekennzeichnet, daß:**
- ein inneres Teil (1) dazu angepaßt ist, in dem Herzhohlraum zu liegen,
- ein äußeres Teil (2) dazu angepaßt ist, außerhalb der Herzwand zu liegen,
- eine Röhre (4, 11) der Öffnung der Herzwand entspricht und der Gefäßprothese als Stütze dient,
- die zwei Teile (1, 2), von denen jedes mit Einrichtungen zum Verhaken (5, 8) an der Herzwand versehen ist, die Herzwand durchdringen,
- das innere Teil (1) sich von der Röhre unterscheidende Einrichtungen (13) zur Führung und zur Befestigung des äußeren Teils (2) trägt,
- die Röhre durch das innere Teil (1) oder durch das äußere Teil (2) getragen wird.

2. Vorrichtung nach Anspruch 1,
**dadurch gekennzeichnet, daß**
das innere Teil (1) die Form eines Kranzes (3) hat und seine Einrichtungen zum Verhaken die Form von Klauen (5) haben.

3. Vorrichtung nach Anspruch 2,
**dadurch gekennzeichnet, daß**
sich die Klauen radial rings um den Kranz (3) erstrecken und jede einen Puffer (6) zur Auflage auf die Herzwand trägt.

4. Vorrichtung nach Anspruch 2,
**dadurch gekennzeichnet, daß**
die Klauen (5) ein Netz aus bioverträglichem Gewebe tragen.

5. Vorrichtung nach Anspruch 2,
**dadurch gekennzeichnet, daß**
die Klauen (5) zum Einführen des inneren Teils (1) in den Herzhohlraum in dem Raum des Kranzes (3) zusammendrückbar sind.

6. Vorrichtung nach Anspruch 2,
**dadurch gekennzeichnet, daß**
die Röhre (4) mit dem Kranz (3) verbunden ist.

7. Vorrichtung nach Anspruch 1,
**dadurch gekennzeichnet, daß**
das äußere Teil (2) die Form einer Platte (7) hat, und seine Einrichtungen zum Verhaken senkrecht zu der Platte (7) ausgerichtete Nadeln (8) sind.

8. Vorrichtung nach Anspruch 7,
**dadurch gekennzeichnet, daß**
die Platte (7) die Röhre (11) trägt.

9. Vorrichtung nach Anspruch 1,
**dadurch gekennzeichnet, daß**
die Einrichtungen zur Führung und zur Befestigung des äußeren Teils (2) aus Stiften (13) gebildet sind, die geeignet sind, vorzugsweise durch Falzen geklemmt und nach der Anbringung der Vorrichtung auf der Herzwand abgetrennt zu werden.

10. Vorrichtung nach Anspruch 2,
**dadurch gekennzeichnet, daß**
die mittige Öffnung des Kranzes (3) während der Anbringung der Vorrichtung durch einen festen oder aufblasbaren, lösbaren Stopfen (14) verschlossen ist.
